# EUROPÄISCHE PATENTSCHRIFT

(11) **EP 1 246 611 B1**
(45) Veröffentlichungstag und Bekanntmachung des Hinweises auf die Patenterteilung: **19.09.2007**
(21) Anmeldenummer: 01915129.9
(22) Anmeldetag: 10.01.2001
(51) Int. Cl.: A61K 9/16, A61K 9/14

(54) **FORMULIERUNGEN VON WIRKSTOFFEN IN FORM EINER FESTEN DISPERSION**
FORMULATIONS OF ACTIVE COMPONENTS IN THE FORM OF A SOLID DISPERSION
FORMULATIONS DE PRINCIPES ACTIFS SE PRESENTANT SOUS LA FORME D'UNE DISPERSION SOLIDE

(30) Priorität: 11.01.2000 DE 10000792
(43) Veröffentlichungstag der Anmeldung: 09.10.2002
(73) Patentinhaber: Abbott GmbH & Co. KG, 65205 Wiesbaden (DE)
(72) Erfinder: LIPPOLD, Bernhard, Universitätsstrasse 1, 40225 Düsseldorf (DE); URBANETZ, Nora, Anne, Universitätsstrasse 1, 40225 Düsseldorf (DE)
(74) Vertreter: Kinzebach, Werner
(86) Internationale Anmeldenummer: PCT/EP2001/000224
(87) Internationale Veröffentlichungsnummer: WO 2001/051025

(56) Entgegenhaltungen:
- EP-A- 0 232 877
- WO-A-93/11749
- US-A- 4 289 749
- NOZAWA, Y. ET AL: "Improving dissolution rate of practically insoluble drug kitasamycin by forcibly roll mixing with additives" PHARM. IND. (1986), 48(8), 967-9 , XP001040986

## Beschreibung

Gegenstand der vorliegenden Erfindung sind lösungsmittelfreie Formulierungen von Wirkstoffen als feste Dispersion, wobei die feste Dispersion aus mindestens einem moleculardispers verteilten Wirkstoff und einem Trägermittel gebildet wird, ein Trägermittel aus Polyvinylpyrrolidon (PVP) und Polyethylenglykol (PEG), ein lösungsmittelfreies Verfahren zur Herstellung der erfindungsgemäßen Formulierungen sowie die Verwendung von geschmolzenem PEG zur Lösung von PVP.

Die schlechte Wasserlöslichkeit vieler neuer Wirkstoffe bringt in zunehmendem Maße Bioverfügbarkeitsprobleme mit sich. Schlechte Wasserlöslichkeit bedeutet nämlich in der Regel ebenfalls die schlechte Löslichkeit des Wirkstoffes in der wässrigen gastrointestinalen Flüssigkeit. Da die Auflösung des Wirkstoffes aber die Voraussetzung für seine Resorption in den Blutkreislauf darstellt, erweisen sich Wirkstoffe mit schlechter Wasserlöslichkeit und damit einhergehender geringer Lösungsgeschwindigkeit als kritisch.

Das Ziel der Arzneiformulierung muss in diesen Fällen die Verbesserung der Lösungseigenschaften in der gastrointestinalen Flüssigkeit sein. Die Lösungsgeschwindigkeit des Wirkstoffes ist entsprechend dem Gesetz nach Noyes und Whitney der benetzten Wirkstofffläche und der Wirkstofflöslichkeit im Gastrointestinal-Trakt proportional.

Beide Parameter können durch die Einbettung des Wirkstoffes in ein inertes, gut wasserlösliches Trägermittel günstig beeinflusst werden. Die Produkte heißen feste Dispersionen. Der Dispersitätsgrad des Wirkstoffs in der Dispersion bestimmt seine Teilchengröße und damit seine Oberfläche, der Kristallinitätsgrad seine Löslichkeit in wässrigen Medien. Ferner kann das hydrophile Trägermittel die Benetzung verbessern.

Nach dem Stand der Technik werden feste Dispersionen durch Sprüheinbettung, Schmelzextrusion oder Schmelzeinbettung gewonnen.

Daneben kommen auch kombinierte Verfahren zur Anwendung.

Die Herstellung von Sprüheinbettungen erfolgt durch Lösen des meist kristallinen Wirkstoffs mit dem Träger in einem gemeinsamen organischen Lösungsmittel. In diesem Stadium der Herstellung liegen sowohl Arzneistoff als auch Träger nicht mehr in einem Molekülverband vor, sondern sind molekulardispers in der Lösung verteilt. Durch Versprühen der Lösung in einem Sprühturm und Abdampfen des Lösungsmittels entsteht ein Kopräzipitat aus Wirkstoff und Träger, wobei der Wirkstoff in Abhängigkeit von den Eigenschaften beider Komponenten molekulardispers im Träger erhalten bleiben, aber auch amorph oder wieder kristallin ausfallen kann. Typisches Trägermittel für Sprüheinbettungen ist Polyvinylpyrrolidon.

Die Nachteile des Verfahrens nach dem Stand der Technik sind insbesondere der sehr hohe apparative Aufwand für das Versprühen der Lösung und die anschließende Lösungsmittelrückgewinnung, die aus Gründen des Umweltschutzes, aber auch aus Gründen der Toxizität und der Lagerstabilität des Endproduktes vollständig sein muss. Der Vorteil des Verfahrens zeigt sich darin, dass aufgrund der rekristallisationsinhibierenden Eigenschaften des Polyvinylpyrrolidons der Wirkstoff bevorzugt in molekulardisperser oder amorpher Form im Träger anfällt und während der Lagerung auch in dieser Form erhalten bleibt. Dies sichert im Vergleich zu kristallin anfallenden Wirkstoffen eine erhöhte Löslichkeit und damit Lösungsgeschwindigkeit des Wirkstoffes in wässrigen Medien.

Unter Umgehung der Lösungsmittelproblematik werden Schmelzextrudate durch Plastifizierung des Trägermittels mit dem Wirkstoff und anschließende Formgebung der plastischen Masse hergestellt. Typisches Trägermittel für Schmelzextrudate ist Polyvinylpyrrolidon.

Soll der Wirkstoff bei diesem Verfahren molekulardispers oder amorph im Trägermittel anfallen, so muss der kristalline Wirkstoff im Verlauf der Herstellung zunächst einmal in den molekulardispersen Zustand überführt werden. Dies gelingt nur dann, wenn sich der Wirkstoff im Trägermittel lösen kann. Häufig ist hierfür eine nicht unerhebliche Wärmezufuhr nötig, die gegebenenfalls bis zum Erreichen des Schmelzens mindestens einer der Komponenten führt. Wirkstoffe, die sich nur lösen, wenn alle Komponenten geschmolzen sind, können nicht mehr extrudiert werden, weil das Material dann flüssig ist und seine plastischen Eigenschaften verloren hat. In allen anderen Fällen, in denen der Wirkstoff im Verlauf der Herstellung nicht in den molekulardispersen Zustand gebracht werden kann, bleibt seine ursprüngliche Kristallinität erhalten und die Löslichkeit in wässrigen Medien ist im Vergleich zu Endprodukten, die den Wirkstoff amorph enthalten, schlechter. Der apparative Aufwand zur Herstellung von Schmelzextrudaten ist sehr hoch.

Die Gewinnung von Schmelzeinbettungen erfolgt durch Aufschmelzen des Trägermaterials und Lösen des Wirkstoffes in der Schmelze und anschließendes Abkühlen dieser Lösung. Typisches Trägermittel ist Polyethylenglykol.

Das Verfahren zeichnet sich im Vergleich zu den beiden obengenannten Methoden durch niedrigsten apparativen Aufwand aus. Die Herstellung gewährleistet im Gegensatz zur Schmelzextrusion zunächst die Überführung des Wirkstoffes in den molekulardispersen Zustand, Voraussetzung für das Vorliegen des Wirkstoffes in molekulardispersem oder amorphem Zustand im Endprodukt. Dabei ist die thermische Belastung wegen des niedrigen Schmelzpunktes des Polyethylenglykols - je nach Molekulargewicht zwischen 35°C und 60°C - und seines im geschmolzenen Zustand guten Lösungsvermögens für viele Wirkstoffe in aller Regel niedrig. Allein die geringeren rekristallisationsinhibierenden Eigenschaften des Polyethylenglykols im Vergleich zu Polyvinylpyrrolidon und seine starke Neigung selbst zu kristallisieren bedingen, dass der Wirkstoff während der Lagerung leicht rekristallisieren kann.

Stellvertretend für eine typische Art der Formulierung von Wirkstoffen gemäß dem Stand der Technik in Form einer festen Dispersion wird die WO-A-90/06115 herangezogen. Die WO-A-90/06115 betrifft Zubereitungen des Oxipurinols und/oder seiner Alkali- oder Erdalkalisalze in nicht kristalliner Form enthaltend den Wirkstoff in Form einer Feststoffdispersion mit pharmakologisch unbedenklichen Hilfsstoffen. Sie weisen eine raschere Lösungsgeschwindigkeit und höhere Löslichkeit auf als Oxipurinol und/oder seine Alkali- oder Erdalkalisalze. Sie können verwendet werden zur Herstellung von Arzneimitteln mit hoher Bioverfügbarkeit des Wirkstoffes Oxipurinol. Diese Zubereitungen können auch weitere Wirkstoffe enthalten oder mit diesen vermischt vorliegen. Die Herstellung der beschriebenen Zubereitungen erfolgt zum Beispiel dadurch, dass Oxipurinol und/oder seine Alkali- oder Erdalkalisalze zusammen mit den Hilfsstoffen gelöst werden oder in einer Hilfsstoffschmelze aufgelöst werden und die erhaltenen Schmelzen oder Lösungen abgekühlt und/oder eingetrocknet werden. Als Lösungsmittel wird vorzugsweise Wasser verwendet.

Als Hilfsstoffe kommen solche in Frage, die entweder aufgeschmolzen oder mit Hilfe eines Lösungsmittels gelöst werden können. Werden derartige Schmelzen oder Lösungen rasch abgekühlt und/oder eingetrocknet, so entstehen Feststoffdispersionen oder sogenannte "feste Lösungen", in denen das Oxipurinol in nicht-kristalliner Form vorliegt.

Die Herstellung der dort beschriebenen Zubereitungen erfolgt beispielsweise dadurch, dass der Wirkstoff und die Hilfsstoffe miteinander aufgeschmolzen und durch Ausgießen zu Platten, Abtropfen auf gekühlte Unterlagen zu Perlen, Eingießen in vorgezogene Blisternäpfe oder Sprüherstarrung abgekühlt werden. Diese Schmelzen können gegebenenfalls auch direkt in Kapseln eindosiert werden.

Die Herstellung der Lösungen kann beispielsweise dadurch erfolgen, dass zunächst nur der Wirkstoff in dem Lösungsmittel vorgelöst wird und dann eine Lösung der Hilfsstoffe zugemischt wird. Es ist auch möglich, den Wirkstoff nachträglich in eine Lösung des Hilfsmittels einzubringen. Das Entfernen des Lösungsmittels erfolgt anschließend durch Verdunsten, Verdampfen im Vakuum, durch Sprühtrocknung oder Gefriertrocknung.

Als Hilfsstoffe zur Herstellung der Oxipurinol-Feststoffdispersionen werden Polyethylenglykole mit mittleren Molekulargewichten von 200 bis etwa 35.000, Polyvinylpyrrolidon (z.B. Kollidon^{®} 17, 25, 30, 90), Polyvinylacetat, Mischpolymerisate aus Polyvinylpyrrolidon/Polyvinylacetat (z.B. Kollidon^{®} VA 64), Polyvinylalkohole unterschiedlichster Verseifungsgrade, Cellulosederivate wie Natriumcarboxymethylcellulose, Hydroxyethylcellulose, Hydroxypropylmethylcellulose, Acrylsäurederivate wie Polyacrylsäure (z.B. Carbopol^{®}) allein oder als Mischung eingesetzt. Besonders bevorzugte Hilfsstoffe sind Polyethylenglykole und Polyvinylpyrrolidone.

Das Dokument EP-A-232877 offenbart die Extrusion einer Zusammensetzung enthaltend PVP und mehrere PEG.

Das der Erfindung zugrunde liegende technische Problem bestand darin, ein möglichst einfaches, kostengünstiges Verfahren ohne hohen apparativen Aufwand bereitzustellen, welches unter Umgehung der Lösungsmittelproblematik aber gleichzeitig niedriger thermischer Belastung des Materials die Herstellung von Wirkstoffformulierungen in Form einer festen Dispersion ermöglicht. Die Formulierungen sollten günstige Freisetzungseigenschaften des Wirkstoffs besitzen und dieselben während der Lagerung beibehalten. Eine Rekristallisation von Wirkstoffen sollte ebenfalls unterdrückt werden.

Das technische Problem wird gelöst durch Formulierungen von Wirkstoffen als feste Dispersion, wobei die feste Dispersion aus mindestens einem Wirkstoff und einem Trägermittel gebildet wird, dadurch gekennzeichnet, dass das Trägermittel eine Mischung von Polyvinylpyrrolidon (PVP) mit einem Gewichtsmittel des Molekulargewichts ≤ 1 500 000 Da und einem bei Temperaturen von 17 - 22°C halbfesten oder festen Polyethylenglykol (PEG) mit einer mittleren molaren Masse von 950 - 3 300 Da ist.

Die erfindungsgemäß getroffene Auswahl von PEG und PVP zur Erlangung der erfindungsgemäßen Formulierungen gehen aus WO-A-90/06115 nicht hervor. Es wurde überraschenderweise gefunden, dass sich Polyvinylpyrrolidon in geschmolzenem Polyethylenglykol löst. Dies erscheint ungewöhnlich, da die Mischbarkeit von Polymeren miteineinander wegen des sehr geringen Entropiegewinns energetisch ungünstig ist.

Außerdem wurde gefunden, dass der Zusatz von Polyvinylpyrrolidon die Lagerstabilität von Schmelzeinbettungen auf der Basis von Polyethylenglykol mit amorphem Wirkstoff zu gewährleisten vermag.

Die derzeit im Handel befindlichen Produkte PEG 1000, PEG 1350, PEG 2000 und PEG 3000 werden durch ihre mittleren molaren Massen charakterisiert (Polyethylenglykole, Produktbeschreibung; Hoechst Aktiengesellschaft, Frankfurt am Main 1992.). Die mittlere molare Masse des PEG 1000 liegt beispielsweise zwischen 950 und 1050 Da, die des PEG 3000 zwischen 2700 und 3300 Da. Die entsprechende Molmassenverteilung um die mittlere molare Masse folgt der Poissonschen Formel (Polyethylenglykole, Produktbeschreibung; Hoechst Aktiengesellschaft, Frankfurt am Main 1992).

Gleichermaßen erfolgt die Charakterisierung der Handelsprodukte des PVP mit K-Werten von 12, 17, 25, 30 und 90 über das Gewichtsmittel des Molekulargewichts (Bühler V.: Kollidon^{®} Polyvinylpyrrolidone for the pharmaceutical industry; BASF Aktiengesellschaft Feinchemie, Ludwigshafen 1993). Dieses beträgt für PVP K12 beispielsweise 2 000 bis 3 000 Da, für PVP K90 1 000 000 bis 1 500 000 Da. Die entsprechende Molekulargewichtsverteilung um das Gewichtsmittel des Molekulargewichts entspricht im Idealfall einer Gaußschen Glockenkurve (Bühler V.: Kollidon^{®} Polyvinylpyrrolidone for the pharmaceutical industry; BASF Aktiengesellschaft Feinchemie, Ludwigshafen 1993).

Diese herstellungsbedingte breite Spezifikation bezüglich der Molmasse von PEG bzw. des Molekulargewichts von PVP kann im Extremfall die Löslichkeit des PVP in PEG beeinflussen oder den Zeitbedarf für die Auflösung des PVP in PEG verändern. Dabei steigt die Löslichkeit des PVP in PEG mit sinkendem Molekulargewicht des PVP und sinkender Molmasse des PEG.

Vorzugsweise beträgt das Gewichtsverhältnis von PEG _{:} PVP bei den erfindungsgemäßen Formulierungen mindestens 0,25, bevorzugt 0,25 - 19, insbesondere 1,5 bis 9. In einer weiteren bevorzugten Ausführungsform beträgt das Gewichtsverhältnis von PEG : PVP mindestens 1,0 vorzugsweise 1,0 - 19, insbesondere 1,5 bis 9.

Vorzugsweise beträgt das Gewichtsmittel des Molekulargewichtes von PVP ≤ 4 000 Da, insbesondere ≤ 34 000 Da oder ≤ 1 000 Da.

Die mittlere molare Masse des PEG liegt bei den erfindungsgemäßen Formulierungen vorzugsweise bei Werten ≤ 2 200 Da.

Als Wirkstoff, der in der erfindungsgemäßen Formulierung verwendet werden kann, kommt grundsätzlich jede Substanz in Betracht, die mit der Umgebung in eine Wechselwirkung tritt und eine mittelbar oder unmittelbar messbare Veränderung der Umgebung bewirkt.

Insbesondere ist der Wirkstoff ein Arzneistoff, Prodrug, kosmetisches Mittel, Nahrungsergänzungsmittel, Pflanzenschutzmittel, Herbizid, Pestizid, Schädlingsbekämpfungsmittel.

Gegenstand der Erfindung ist auch ein Trägermittel aus PVP mit einem Gewichtsmittel des Molekulargewichtes ≤ 1 500 000 Da und einem bei Temperaturen von 17 - 22°C halbfesten oder festen Polyethylenglykol (PEG) mit einer mittleren molaren Masse von 950 - 3 300 Da. Dieses Trägermittel kann als Zwischenprodukt auf dem Weg der Herstellung einer erfindungsgemäßen Formulierung eingesetzt werden, etwa indem das erfindungsgemäße Trägermittel aufgeschmolzen und mit dem Wirkstoff versetzt wird.

Gegenstand der Erfindung ist ebenfalls ein lösungsmittelfreies Verfahren zur Herstellung der erfindungsgemäßen Formulierungen. Dabei werden PEG und PVP mit Wirkstoff versetzt und gemeinsam in einem Verfahrensschritt aufgeschmolzen oder PVP wird in geschmolzenem PEG gelöst, danach wird der Wirkstoff zugefügt, wobei eine molekulardisperse Verteilung des Wirkstoffs erreicht wird. Nach Abkühlung kann dann die erfindungsgemäße Wirkstoffformulierung erhalten werden.

Die mittels des erfindungsgemäßen Verfahrens erhältlichen WirkstoffFormulierungen weisen überraschenderweise eine hohe Lagerstabilität auf.

Vorzugsweise wird erfindungsgemäß zur Herstellung der festen Dispersion ein bei Temperaturen von 17 - 22°C halbfestes oder festes Polyethylenglykol (PEG) mit einer mittleren molaren Masse von 950 - 3 300 Da verwendet. PVP wird vorzugsweise mit einem Gewichtsmittel des Molekulargewichts von < 1 500 000 Da eingesetzt.

Gegenstand der Erfindung ist ferner die Verwendung von einem bei Temperaturen von 17 - 22°C halbfesten oder festen Polyethylenglykol (PEG) mit einer mittleren molaren Masse von 950 - 3 300 Da in geschmolzenem Zustand zur Lösung von PVP mit einem Gewichtsmittel des Molekulargewichts von ≤ 500 000 Da.

Die Erfindung wird anhand der folgenden Beispiele näher erläutert.

### Beispiele 1 und 2: Herstellung und Eigenschaften von Formulierungen

| Beispiel 1: | | Beispiel 2: | |
|---|---|---|---|
| Nimodipin | 20% | Nimodipin | 20% |
| PVP K 17 | 32% | PVP K 17 | 16% |
| PEG 2 000 | 48% | PEG 2 000 | 64% |

Die Herstellung fester Dispersionen eines Wirkstoffes mit einer Mischung aus Polyethylenglykol 2 000 (mittlere molare Masse 1 800 - 2 200 Da) und Polyvinylpyrrolidon K 17 (Gewichtsmittel des Molekulargewichts 7 000 - 11 000) erfolgt durch Schmelzeinbettung. Hierfür wurde zunächst Polyethylenglykol 2 000 geschmolzen und anschließend zuerst Polyvinylpyrrolidon K 17 und danach Nimodipin als Wirkstoff in der Schmelze gelöst. Die Lösung wurde in Formen ausgegossen. Die Untersuchung des Freisetzungsverhaltens erfolgte nach einem Tag und 4 bzw. 24 Wochen Lagerung.

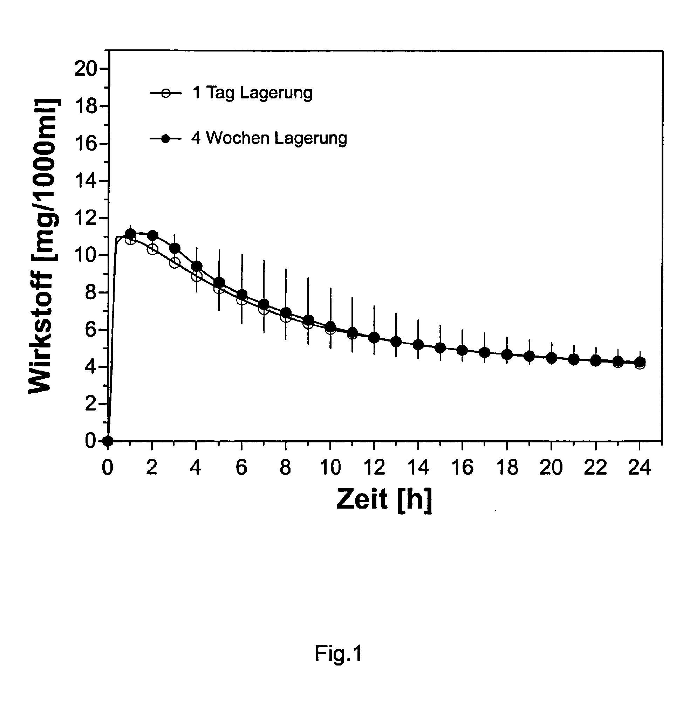

Figur 1 zeigt das Freisetzungsprofil von festen Dispersionen aus 20 Gew.% Nimodipin, 32 Gew.% Polyvinylpyrrolidon K 17 und 48 Gew.% Polyethylenglykol 2 000 (Beispiel 1) nach einem Tag und 4 Wochen Lagerung. Es ist charakterisiert durch eine hohe Freisetzungsgeschwindigkeit und eine Übersättigung des Wirkstoffes im Freisetzungsmedium. Diese Freisetzungscharakteristik ist nach 4 Wochen Lagerung unverändert.

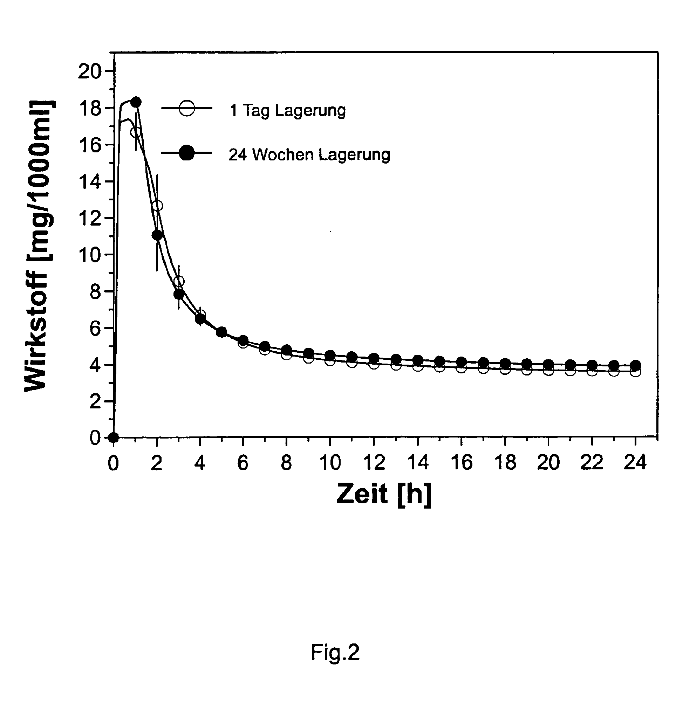

Figur 2 zeigt das Freisetzungsprofil von Dispersionen aus 20% Gew.% Nimodipin, 16% Gew.% Polyvinylpyrrolidon K 17 und 64 Gew.% Polyethylenglykol 2 000 (Beispiel 2) nach einem Tag und 24 Wochen Lagerung. Es ist charakterisiert durch eine hohe Freisetzungsgeschwindigkeit und eine extrem hohe Übersättigung des Wirkstoffes im Freisetzungsmedium. Diese Freisetzungscharakteristik ist nach 24 Wochen Lagerung unverändert.

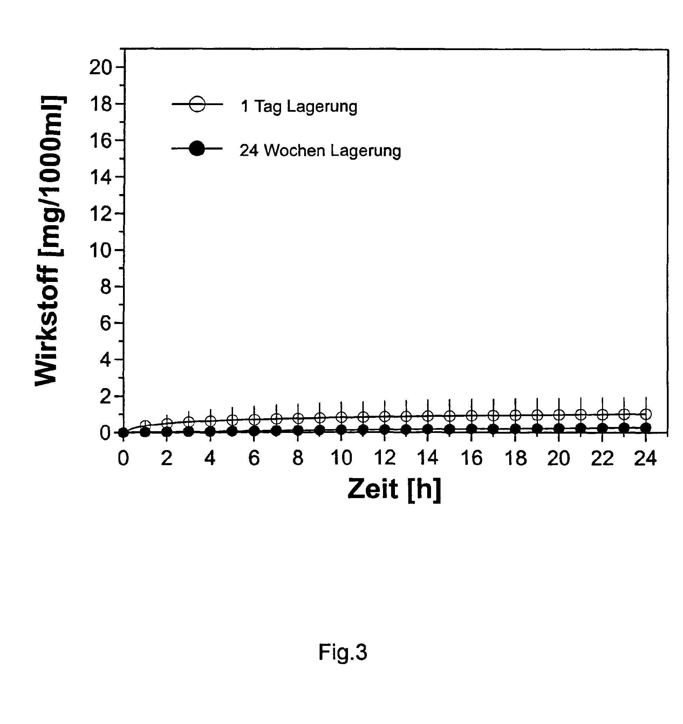

Demgegenüber demonstriert Figur 3 das Freisetzungsverhalten von festen Dispersionen ohne Polyvinylpyrrolidon (80 Gew.% PEG 2 000 und 20 Gew.% Nimodipin). Die Freisetzungsgeschwindigkeit ist bereits nach eintägiger Lagerung im Vergleich zu polyvinylpyrrolidonenthaltenden Präparaten deutlich geringer und verschlechtert sich nach 24 Wochen Lagerung abermals.

Röntgendiffraktometrische Untersuchungen belegen, dass im Falle von polyvinylpyrrolidonfreien Produkten der Wirkstoff nach Lagerung kristallin vorliegt, wohingegen er bei polyvinylpyrrolidonhaltigen Produkten in amorpher Form erhalten bleibt:

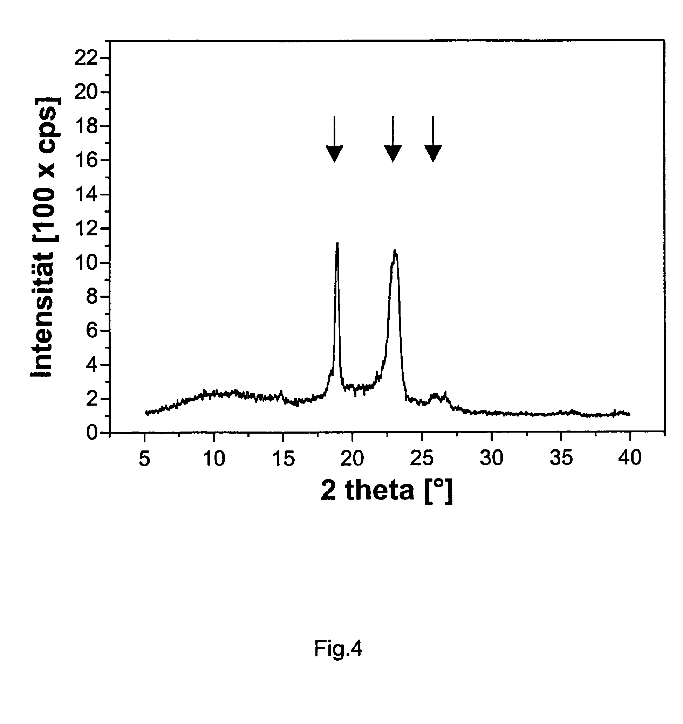

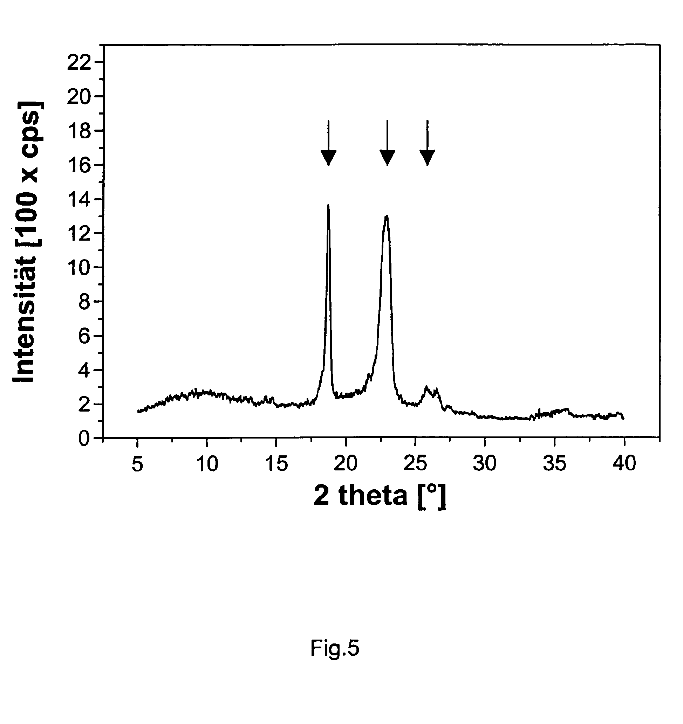

Figur 4 zeigt das Diffraktogramm von festen Dispersionen aus 20 Gew.% Nimodipin, 32 Gew.% Polyvinylpyrrolidon K 17 und 48 Gew.% Polyethylenglykol 2 000 (Beispiel 1) nach 4 Wochen Lagerung, Figur 5 das Diffraktogramm von Dispersionen aus 20% Gew.% Nimodipin, 16% Gew.% Polyvinylpyrrolidon K 17 und 64 Gew.% Polyethylenglykol 2 000 (Beispiel 2) nach 24 Wochen Lagerung. Die Pfeile deuten jeweils auf die Signale des kristallinen Polyethylenglykols. Anzeichen von kristallinem Wirkstoff fehlen.

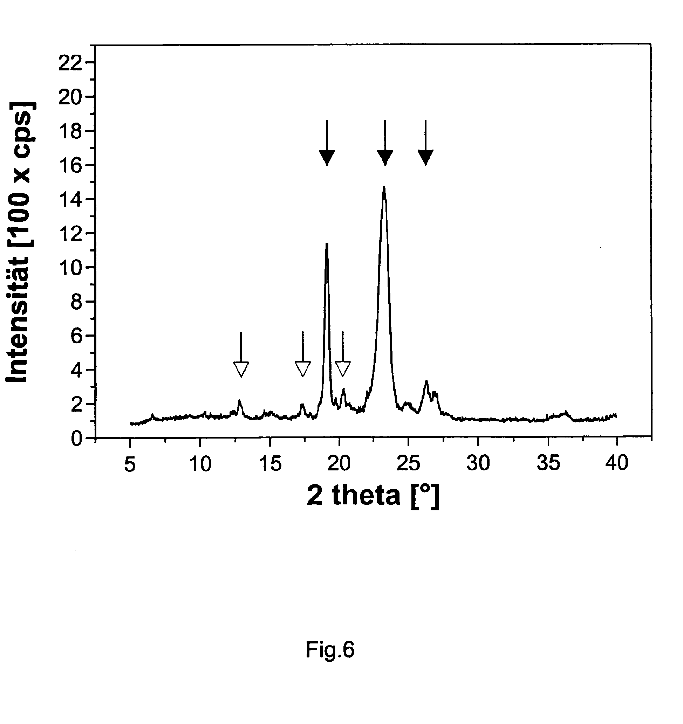

Im Gegensatz dazu treten in der Figur 6, die das Diffraktogramm der polyvinylpyrrolidonfreien Präparate nach 24 Wochen Lagerung zeigt, zu den Signalen des kristallinen Polyethylenglykols Anzeichen von kristallinem Wirkstoff hinzu. Diese sind durch Pfeile mit offenen Spitzen gekennzeichnet.

Die Ergebnisse beweisen, daß der Zusatz von Polyvinylpyrrolidon zu festen Dispersionen auf der Basis von Polyethylenglykol die Rekristallisation des amorphen Wirkstoffes aus dem Endprodukt zu verhindern vermag. Polyvinylpyrrolidon garantiert dadurch ausgezeichnete Lagerstabilität der Polyethylenglykolschmelzeinbettung und gleichbleibend hervorragendes Freisetzungsverhalten.

Durch das erfindungsgemäße Verfahren ist es nunmehr möglich geworden, die Vorteile der konventionellen Schmelzeinbettung auf der Basis von Polyethylenglykol zu nützen, ohne den Nachteil der Lagerinstabilität in Kauf nehmen zu müssen.

Im Vergleich zu bekannten Technologien zeichnet sich das erfindungsgemäße Verfahren durch seine universellen Einsatzmöglichkeiten aus. Aufgrund des guten Lösungsvermögens des geschmolzenen Polyethylenglykols für viele Wirkstoffe bleibt die Anwendung nicht auf Einzelfälle beschränkt. Das robuste Verfahren erlaubt mit vergleichsweise niedrigem technischen Aufwand, unter Umgehung der Problematik organischer Lösungsmittel und hoher thermischer Belastung, die Herstellung von Formulierungen, insbesondere Arzneiformen mit hervorragenden Freisetzungseigenschaften und ausgezeichneter Lagerstabilität.

### Beispiele 3, 4 und 5: Herstellung von Trägermitteln

| Beispiel 3: | | |
|---|---|---|
| PVP K 12 | 80% | (mittlere molare Masse 2 000 - 3 000 Da) |
| PEG 1 000 | 20% | (mittlere molare Masse 950 - 1 050 Da) |

| Beispiel 4: | | |
|---|---|---|
| PVP K 90 | 60% | (mittlere molare Masse 1 000 000 - 1 500 000 Da) |
| PEG 1 000 | 40% | (mittlere molare Masse 950 - 1 050 Da) |

| Beispiel 5: | | |
|---|---|---|
| PVP K 12 | 5% | (mittlere molare Masse 2 000 - 3 000 Da) |
| PEG 3 000 | 95% | (mittlere molare Masse 2 700 - 3 300 Da) |

Die Herstellung der Trägermittel aus Polyethylenglykol und Polyvinylpyrrolidon erfolgte durch Schmelzen des Polyethylenglykols und anschließendes Lösen des Polyvinylpyrrolidons. Die Lösung wurde abgekühlt und erstarrte zum festen Trägermittel.

## Patentansprüche

1. Lösungsmittelfreie Formulierungen von Wirkstoffen als feste Dispersion, wobei die feste Dispersion aus mindestens einem molekulardispers verteilten Wirkstoff und einem Trägermittel gebildet wird, wobei das Trägermittel eine Mischung von Polyvinylpyrrolidon (PVP) mit einem Gewichtsmittel des Molekulargewichts ≤ 1 500 000 Da und einem Polyethylenglykol-Bestandteil (PEG) ist, der aus bei Temperaturen von 17-22 °C halbfestem oder festem PEG mit einer mittleren molaren Masse von 950-3300 Da besteht.

2. Formulierungen nach Anspruch 1, **dadurch gekennzeichnet, dass** das Gewichtsverhältnis von PEG : PVP mindesten 0,25, vorzugsweise 0,25 bis 19, insbesondere 1,5 bis 9 beträgt.

3. Formulierungen nach Anspruch 1, **dadurch gekennzeichnet, dass** das Gewichtsverhältnis von PEG : PVP mindestens 1,0, vorzugsweise 1,0 bis 19, insbesondere 1,5 bis 9 beträgt.

4. Formulierungen nach mindestens einem der Ansprüche 1 bis 3, **dadurch gekennzeichnet, dass** das Gewichtsmittel des Molekulargewichtes von PVP ≤ 54000 Da, vorzugsweise ≤ 34000 Da oder ≤ 11000 Da beträgt.

5. Formulierungen nach mindestens einem der Ansprüche 1 bis 4, **dadurch gekennzeichnet, dass** die mittlere molare Masse des PEG ≤ 2200 Da ist.

6. Formulierungen nach mindestens einem der Ansprüche 1 bis 5, **dadurch gekennzeichnet, dass** der Wirkstoff eine Substanz ist, die mit der Umgebung in eine Wechselwirkung tritt und eine mittelbar oder unmittelbar messbare Veränderung der Umgebung bewirkt.

7. Formulierungen nach Anspruch 6, **dadurch gekennzeichnet, dass** der Wirkstoff ein Arzneistoff, Prodrug, kosmetisches Mittel, Nahrungsergänzungsmittel, Pflanzenschutzmittel, Herbizid, Pestizid, Schädlingsbekämpfungsmittel ist.

8. Lösungsmittelfreies Verfahren zur Herstellung von Formulierungen nach mindestens einem der Ansprüche 1 bis 7, **dadurch gekennzeichnet, dass** PEG und PVP mit Wirkstoff versetzt werden und gemeinsam in einem Verfahrensschritt geschmolzen werden oder PVP in geschmolzenem PEG gelöst wird, danach der Wirkstoffzugefügt wird, wobei eine molekulardisperse Verteilung des Wirkstoffs erreicht wird und nach Abkühlung eine Wirkstoffformulierung erhalten wird.

9. Formulierung nach mindestens einem der Ansprüche 1 bis 7, erhältlich durch ein Verfahren gemäß Anspruch 8.

## Claims

1. Solvent-free formulations of active components as solid dispersion, wherein the solid dispersion is formed from at least one molecularly dispersed active component and one carrier, **characterized in that** the carrier is a mixture of polyvinylpyrrolidone (PVP) with a weight average molecular weight of ≤ 1 500 000 Da and of a polyethylene glycol (PEG) component, the PEG component consisting of PEG which is semisolid or solid at temperatures of 17 - 22 °C and has an average molar mass of 950 - 3 300 Da.

2. Formulations as claimed in claim 1, **characterized in that** the PEG : PVP ratio by weight is at least 0.25, preferably 0.25 to 19, in particular 1.5 to 9.

3. Formulations as claimed in claim 1, **characterized in that** the PEG : PVP ratio by weight is at least 1.0, preferably 1.0 to 19, in particular 1.5 to 9.

4. Formulations as claimed in at least one of claims 1 to 3, **characterized in that** the weight average molecular weight of PVP is ≤ 54 000 Da, preferably ≤ 34 000 Da or ≤ 11 000 Da.

5. Formulations as claimed in at least one of claims 1 to 4, **characterized in that** the average molar mass of PEG is ≤ 2 200 Da.

6. Formulations as claimed in at least one of claims 1 to 5, **characterized in that** the active component is a substance which interacts with the surroundings and brings about a change, which can be measured directly or indirectly, in the surroundings.

7. Formulations as claimed in claim 6, **characterized in that** the active compound is a medicinal substance, prodrug, cosmetic agent, food supplement, crop protection agent, herbicide, pesticide, pest-control agent.

8. A solvent-free process for the production of formulations as claimed in at least one of claims 1 to 7, **characterized in that** PEG and PVP are mixed with active component and melted together in one process step, or PVP is dissolved in molten PEG, and then the active component is added, resulting in a molecular dispersion of the active component and, after cooling, an active component formulation is obtained.

9. A formulation as claimed in at least one of claims 1 to 7, obtained by a process as claimed in claim 8.

## Revendications

1. Formulations sans solvant de principes actifs sous forme de dispersion solide, où la dispersion solide est formée à partir d'au moins un principe actif à répartition de type dispersion moléculaire et d'un support, où le support est un mélange de polyvinylpyrrolidone (PVP) ayant une moyenne en poids du poids moléculaire ≤ 1 500 000 Da et d'un constituant polyéthylèneglycol (PEG), qui consiste en PEG semi-solide ou solide à des températures de 17-22°C ayant une masse molaire moyenne de 950-3300 Da.

2. Formulations selon la revendication 1, **caractérisées en ce que** le rapport en poids PEG:PVP est d'au moins 0,25, de préférence de 0,25 à 19, en particulier de 1,5 à 9.

3. Formulations selon la revendication 1, **caractérisées en ce que** le rapport en poids PEG:PVP est d'au moins 1,0, de préférence 1,0 à 19, en particulier 1,5 à 9.

4. Formulations selon au moins l'une des revendications 1 à 3, **caractérisées en ce que** la moyenne en poids du poids moléculaire de la PVP est ≤ 54 000 Da, de préférence ≤ 34 000 Da ou ≤ 11 000 Da.

5. Formulations selon au moins l'une des revendications 1 à 4, **caractérisées en ce que** la masse molaire moyenne du PEG est ≤ 2200 Da.

6. Formulations selon au moins l'une des revendications 1 à 5, **caractérisées en ce que** le principe actif est une substance qui entre en interaction avec l'environnement et produit une modification de l'environnement qui est mesurable indirectement ou directement.

7. Formulations selon la revendication 6, **caractérisées en ce que** le principe actif est un médicament, un promédicament, un agent cosmétique, un complément alimentaire, un agent de protection des plantes, un herbicide, un pesticide, un agent de lutte contre les nuisibles.

8. Procédé sans solvant pour produire des formulations selon au moins l'une des revendications 1 à 7, **caractérisé en ce que** le PEG et la PVP sont additionnés de principe actif et sont fondus ensemble dans une étape de procédé ou bien la PVP est dissoute dans le PEG fondu, après quoi le principe actif est ajouté, où une répartition du principe actif de type dispersion moléculaire est atteinte et, après refroidissement, une formulation de principe actif est obtenue.

9. Formulation selon au moins l'une des revendications 1 à 7 pouvant être obtenue par un procédé selon la revendication 8.
